# EUROPEAN PATENT APPLICATION

(11) **EP 4 364 654 A1**
(43) Date of publication of application: **08.05.2024**
(21) Application number: 22833322.5
(22) Date of filing: 01.07.2022
(51) Int. Cl.: A61B 5/055, G01N 24/00

(54) **BED FOR FUNCTIONAL MRI AND BED SYSTEM FOR FUNCTIONAL MRI COMPRISING SAME**

(30) Priority: 01.07.2021 JP 2021110184
(71) Applicant: Central Institute For Experimental Animals, Kawasaki-shi, Kanagawa 210-0821 (JP)
(72) Inventor: YURIMOTO Terumi, Kawasaki-shi, Kanagawa 210-0821 (JP); SEKI Fumiko, Kawasaki-shi, Kanagawa 210-0821 (JP); SASAKI Erika, Kawasaki-shi, Kanagawa 210-0821 (JP); INOUE Takashi, Kawasaki-shi, Kanagawa 210-0821 (JP)
(74) Representative: D Young & Co LLP
(86) International application number: PCT/JP2022/026447
(87) International publication number: WO 2023/277177

(57) **Abstract**

The invention provides a bed that enables non-invasive functional MRI for general purposes. The invention provides a functional MRI bed system equipped with a helmet to immobilize the head of the animal with a U-shaped fixture.

## Description

### Technical Field

The present invention relates to the field of functional MRI technology, for example, the field of functional MRI technology for a laboratory animal.

### Background Art

Magnetic resonance imaging (MRI) has extensively been used as a means for measuring activity of the brain. Concerning conventional awake functional MRI for small animals, such as marmosets, mice, and rats, there have been substantially no practical examples of MRI performed using a small-bore MRI apparatus with a bore diameter smaller than 30 cm without surgery.

Previous MRI imaging for awake small animals required surgical operation of the head of the animal to mount an immobilization fixture (e.g., a head post or head bar) thereon so as to immobilize the head of the imaging subject animal. In general, in the present technical field, an immobilization fixture attached in the vertical direction to the head of the animal is referred to as a "head post," and an immobilization fixture attached to the frontal region of the head of the animal is referred to as a "head bar." For example, Non-Patent Literature 1 (Yoshida et al., 2016, Journal of Neuroscience Methods, 274, 38-48) reports functional MRI performed on an awake mouse. However, the head bar is a special-order product, and mounting of the head bar onto the skull is carried out by surgery.

When neither a head bar nor a head post is used, it was necessary to prepare a head immobilization fixture that would fit the subject animal, and there were no fixtures that could be used for general-purpose. For example, although Non-Patent Literature 2 reports functional MRI performed on an awake marmoset in a resting state, such functional MRI is performed using a helmet prepared to be made compatible with each individual animal (Belcher et al., 2013, Journal of Neuroscience, 33 (42), 16796-16804), and it is difficult to recognize such helmet as a general-purpose helmet. In addition, in order to obtain high-resolution images with conventional MRI, use of an MRI apparatus with a high flux density was necessary.

### Citation List

### Non-Patent Literature

Non-Patent Literature 1: Yoshida et al., 2016, Journal of Neuroscience Methods, 274, 38-48
Non-Patent Literature 2: Belcher et al., 2013, Journal of Neuroscience, 33 (42), 16796-16804

### Summary of Invention

### Technical Problem

It is an object of the present invention to provide a small bed that enables non-invasive and general-purpose functional MRI imaging, to solve at least some of the above-described problems. It is another object of the present invention to provide a coil-integrated bed that can achieve a satisfactory resolution of an MRI apparatus.

### Solution to Problem

The present inventors have conducted concentrated studies in order to attain the above objects. As a result, the present inventors discovered that, for example, use of an adjustable U-shaped fixture enables immobilization of the head without surgery and enables non-invasive and general-purpose functional MRI measurement, compared to conventional methods involving use of a head post. Thus, the present invention including the same as an embodiment has been completed.

The present inventors conducted demonstrating experiments using marmosets and found that, when the bore diameter (inner diameter) of an MRI apparatus was relatively small (e.g., 10 cm or smaller), it was not possible to retain sufficient space while providing a receiver coil close to the target to improve the signal-to-noise ratio of the image. The present inventors further conducted concentrated studies and developed a mechanism that enables improved image quality and immobilization of the head of the subject animal by using the receiver coil as a fixture to immobilize the imaging subject. The present inventors also discovered that the use of such MRI apparatus (also referred to as an "improved MRI apparatus") enables awake MRI measurement in a small space because the receiver coil can be provided in a narrow space. Thus, the present invention including the same as an embodiment has been completed.

The present invention encompasses the following embodiments.
[1] A general-purpose functional magnetic resonance imaging (MRI) bed equipped with a bed unit, a collar unit, and a helmet unit,
   wherein
   the bed unit is constituted to hold a laboratory animal in the sphinx position,
   the helmet unit is to immobilize the head of the laboratory animal, so as to perform functional MRI measurement, and the helmet unit comprises an inner helmet comprising a coil (receiver coil) integrated therein and an outer helmet, and
   the collar unit is to restrain the neck and the shoulder of the laboratory animal.
[2] The general-purpose functional MRI bed of embodiment 1, wherein the bed, the helmet, and the collar are made of non-magnetic materials.
[3] The general-purpose functional MRI bed of embodiment 1 or 2, which is used for a marmoset, mouse, rat, macaque, guinea pig, or newborn human.
[4] A general-purpose functional MRI bed equipped with a bed unit, a helmet unit, a collar unit, and a U-shaped fixture,
   wherein
   the bed unit is constituted to hold a laboratory animal in the sphinx position,
   the helmet unit is to immobilize the head of the laboratory animal, so as to perform functional MRI measurement, and the helmet unit comprises a U-shaped fixture-mounting unit to mount a U-shaped fixture,
   the collar unit is to restrain the neck and the shoulder of the laboratory animal, and
   the U-shaped fixture is adjustable in accordance with the dimensions and the configuration of the head of the laboratory animal subjected to functional MRI measurement.
[5] The general-purpose functional MRI bed of embodiment 4, wherein the helmet unit comprises two or more U-shaped fixture-mounting units to mount U-shaped fixtures.
[6] The general-purpose functional MRI bed of embodiment 4, wherein the U-shaped fixture-mounting unit is constituted to mount the U-shaped fixture at a right angle to the longitudinal direction of the MRI bed unit or to mount the U-shaped fixture at an oblique angle to the longitudinal direction of the MRI bed unit; or
   (i) the general-purpose functional MRI bed of embodiment 5, wherein the U-shaped fixture-mounting unit is constituted to mount at least one U-shaped fixture at an oblique angle to the longitudinal direction of the MRI bed unit and to mount at least another U-shaped fixture at a right angle to the longitudinal direction of the MRI bed unit,
   (ii) the general-purpose functional MRI bed of embodiment 5, wherein the U-shaped fixture-mounting unit is constituted to mount at least one U-shaped fixture at a right angle to the longitudinal direction of the MRI bed unit and to mount at least another U-shaped fixture at a right angle to the longitudinal direction of the MRI bed unit, or
   (iii) the general-purpose functional MRI bed of embodiment 5, wherein the U-shaped fixture-mounting unit is constituted to mount at least one U-shaped fixture at an oblique angle to the longitudinal direction of the MRI bed unit and to mount at least another U-shaped fixture at an oblique angle to the longitudinal direction of the MRI bed unit.
[7] The general-purpose functional MRI bed of embodiment 6 dependent on embodiment 4, wherein the U-shaped fixture-mounting unit is constituted to mount a U-shaped fixture at a sharp angle to the longitudinal direction of the MRI bed unit; or
   (i) the general-purpose functional MRI bed of embodiment 6 dependent on embodiment 5, wherein the U-shaped fixture-mounting unit is constituted to mount at least one U-shaped fixture at a sharp angle to the longitudinal direction of the MRI bed unit and to mount at least another U-shaped fixture at a right angle to the longitudinal direction of the MRI bed unit, or
   (ii) the general-purpose functional MRI bed of embodiment 6 dependent on embodiment 5, wherein the U-shaped fixture-mounting unit is constituted to mount at least one U-shaped fixture at a sharp angle to the longitudinal direction of the MRI bed unit and to mount at least another U-shaped fixture at an obtuse angle to the longitudinal direction of the MRI bed unit.
[8] The general-purpose functional MRI bed of any of embodiments 4 to 7, wherein the bed, the helmet, the collar, the U-shaped fixture, and a screw to immobilize the U-shaped fixture are made of non-magnetic materials.
[9] The general-purpose functional MRI bed of any of embodiments 4 to 8, which is used for a marmoset.
[10] A functional MRI bed system equipped with the general-purpose functional MRI bed of any of Embodiments 4 to 9 and an MRI apparatus.
[11] A method of functional MRI measurement for an animal, comprising using the general-purpose functional MRI bed of any of embodiments 4 to 9 or the functional MRI bed system of embodiment 10.
[12] The method of embodiment 11, comprising measuring the brain in a resting state.
[13] The method of functional MRI measurement for an animal of embodiment 11, wherein an imaging device is provided in front of the face of the subject animal of imaging, and the MRI measurement is performed simultaneously with eye tracking.

The description incorporates the contents disclosed in JP Patent Application No. 2021-110184, which is basis for priority of the present application.

### Advantageous Effects of Invention

In the present invention, a functional MRI bed can be used for different individuals.

### Brief Description of Drawings

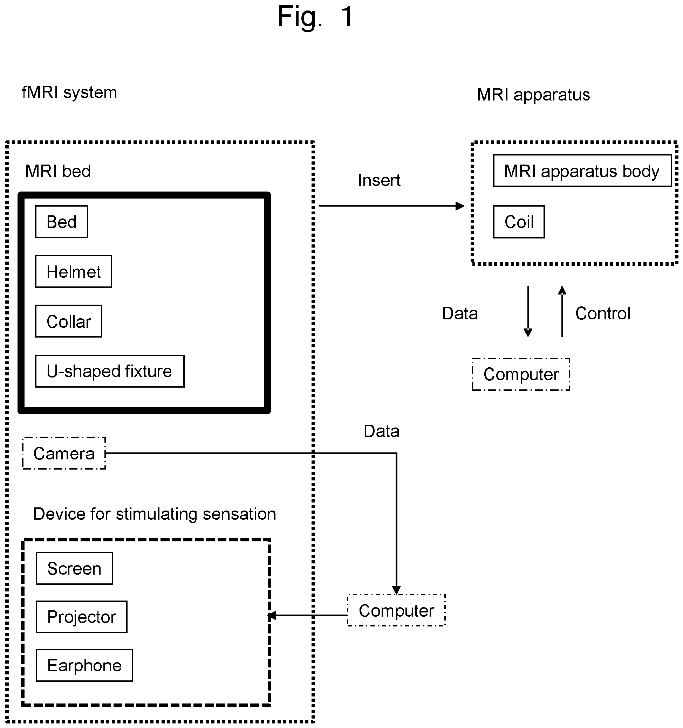

[Figure 1] Figure 1 shows a configuration of a functional MRI bed system.
[Figure 2] Figure 2 shows an elevational view and a plan view of a functional MRI bed (a bed unit).
[Figure 3] Figure 3 shows a 3D-CAD-designed functional MRI bed.
[Figure 4] Figure 4 shows a photograph of a 3D-printed functional MRI bed.
[Figure 5] Figure 5 shows a schematic view (left) and a photograph (right) of the head of an animal immobilized with a U-shaped fixture in a helmet.
[Figure 6] Figure 6 shows functional MRI image and a MR signal waveform of a marmoset obtained using a functional MRI bed system. The upper MR signal indicates the waveform of the prefrontal cortex and the lower MR signal indicates the waveform of the auditory cortex.
[Figure 7] Figure 7 shows the atlas of the marmoset brain.
[Figure 8] Figure 8 shows the superior/inferior, right/left, and anterior/posterior shift lengths of the brain of the target of MRI imaging.

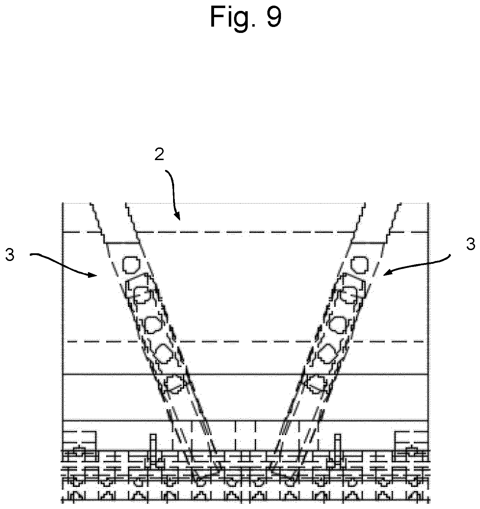

[Figure 9] Figure 9 shows a lateral view showing the state of the U-shaped fixture mounted in a helmet unit. In the configuration shown in the left, the first U-shaped fixture is mounted at a sharp angle to the longitudinal direction of the MRI bed unit and the second U-shaped fixture is mounted at a right angle to the longitudinal direction of the MRI bed unit. In the configuration shown in the right, the first U-shaped fixture is mounted at a sharp angle to the longitudinal direction of the MRI bed unit, and the second U-shaped fixture is mounted at an obtuse angle to the longitudinal direction of the MRI bed unit.
[Figure 10] Figure 10 shows a schematic view of an inner helmet and an outer helmet.
[Figure 11] Figure 11 shows a photograph of an inner helmet comprising a coil body mounted thereon.
[Figure 12] Figure 12 shows a photograph of a coil-mounted inner helmet mounted on an animal.
[Figure 13] Figure 13 shows a photograph showing an inner helmet on which an outer helmet is further mounted. The inner helmet comprises, on the right side and the left side thereof, sponges to immobilize the inner helmet.
[Figure 14] Figure 14 shows the correlation between the photograph of the animal on which a coil body-mounted inner helmet and an outer helmet are mounted (left) and the schematic view (right).
[Figure 15] Figure 15 shows a functional MRI image of a marmoset obtained using a functional MRI bed system (left) and a functional MRI image of a marmoset obtained using an improved MRI bed (right).
[Figure 16] Figure 16 shows the superior/inferior, right/left, and anterior/posterior shift lengths of the brain of the target of MRI imaging obtained using a functional MRI bed system.
[Figure 17] Figure 17 shows the superior/inferior, right/left, and anterior/posterior shift lengths of the brain of the target of MRI imaging obtained using an improved MRI bed.
[Figure 18] Figure 18 shows functional MRI images of a rat obtained using an improved MRI bed. Figure 18 A shows an image obtained using a coil for a marmoset and Figure 18 B shows an image obtained using a single-purpose coil for the rat head.
[Figure 19] Figure 19 shows T2-weighted images of functional MRI of a rat obtained using an improved MRI bed. Figure 19 A shows an image obtained using a coil for a marmoset and Figure 19 B shows an image obtained using a single-purpose coil for a rat head.
[Figure 20] Figure 20 shows a CT image of a marmoset obtained using a standard MRI bed.
[Figure 21] Figure 21 shows an eye tracking image obtained by video recording using another video camera performed at the time of MRI imaging performed using a standard MRI bed. Description of Embodiments

In an embodiment, the present invention provides a functional MRI bed. In another embodiment, the present invention provides a functional MRI bed system equipped with a functional MRI bed. In an embodiment, the functional MRI bed system according to the present invention comprises an awake (awake state) functional MRI system. The functional MRI system comprises an MRI bed. In addition, the functional MRI system can be equipped with a camera and a device for stimulating sensation. When the device for stimulating sensation is to stimulate the visual and auditory senses, the device can be equipped with a screen, a projector, and/or an earphone. The camera can be connected to a computer. Thus, the data can be incorporated into the computer from the camera. Other devices for stimulating sensation can stimulate the olfactory sensation or touch sensation. The computer can control the device for stimulating sensation. It should be noted that the camera, the device for stimulating sensation, and the computer are not essential components, and the external camera, device for stimulating sensation, or computer may be external parts that are connected. The functional MRI system is inserted into the MRI apparatus and controlled by the computer, according to need to carry out MRI measurement. In the present invention, unless specified otherwise, the MRI apparatus comprises the MRI apparatus body and a coil. The functional MRI bed system according to the present invention can be used in a computed tomography machine.

In an embodiment, the functional MRI bed is equipped with a bed unit, a helmet unit, a collar unit, and a U-shaped fixture. The collar unit is to restrain the neck and the shoulder of an animal. The U-shaped fixture is to restrain the head of an animal.

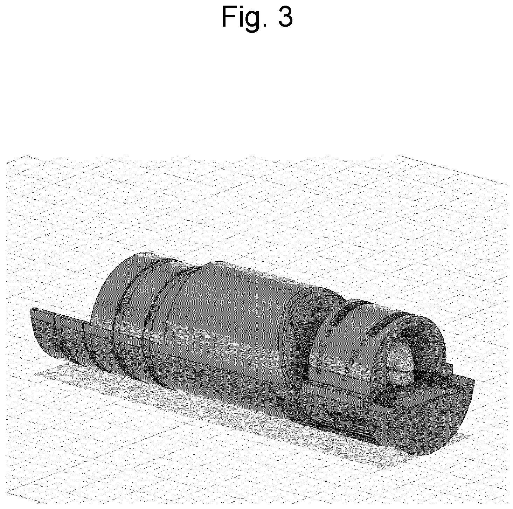

When performing MRI measurement, the functional MRI system is inserted into the MRI apparatus. The coil is placed to perform MRI measurement of the immobilized animal. In an embodiment, the coil has a rolled form, and the coil has dimensions that enables inserting the entire functional MRI bed (e.g., the functional MRI bed shown in Figure 3) into the space inside the coil. In another embodiment, the receiver coil can be integrated into the inner helmet. The MRI apparatus can be controlled by a computer. The measured data obtained using the MRI apparatus are transferred to the computer.

The term "functional MRI bed system" used herein does not refer to only the functional MRI bed included in the system, but rather the term refers to the entire functional MRI bed system equipped with the functional MRI bed and the MRI apparatus body. The term "functional MRI bed" used herein does not refer to only the bed unit included in the functional MRI bed, but rather the term refers to the functional MRI bed equipped with the bed unit, the helmet unit, the collar unit, and the U-shaped fixture. The functional MRI bed may simply be referred to herein as a "retainer". The functional MRI bed equipped with the U-shaped fixture may be referred to herein as a "standard functional MRI bed".

In an embodiment, the diameter of the retainer (functional MRI bed) used for a small-bore MRI with the bore diameter of smaller than 30 cm is preferably 10 cm or smaller. Such retainer can be used, for example, for a marmoset. The MRI bed unit comprises a collar unit to immobilize the shoulder and the neck, a tunnel unit to immobilize the waist part, and a helmet unit to immobilize the head of the animal. The helmet unit is to accommodate the head of an animal. Corresponding to the helmet unit, the bed unit can comprise a chin rest. A region closer to the helmet in the tunnel unit can be equipped with a collar-mounting unit to mount the collar. A part in the tunnel unit on the opposite side from the helmet can be equipped with a corset. The corset can be made of a flexible material such as a sponge. Also, the corset can comprise a zip-tie-mounting unit, so as to restrain the lower body or the hindlimbs of an animal with a zip tie. The corset may immobilize the subject by using an adhesive tape instead of a zip tie. The MRI bed unit can comprise a pocket unit to accommodate the forelimbs when the animal is placed in the sphinx position.

The helmet unit of the standard functional MRI bed can comprise one to four U-shaped fixture-mounting units to mount U-shaped fixtures. In an embodiment, the helmet unit comprise one U-shaped fixture-mounting unit. In another embodiment, the helmet unit comprise two U-shaped fixture-mounting units. In another embodiment, the helmet unit comprises three U-shaped fixture-mounting units. In another embodiment, the helmet unit comprises four U-shaped fixture-mounting units. The U-shaped fixture-mounting unit may be constituted to mount the U-shaped fixture at a right angle to the longitudinal direction of the MRI bed unit or to mount the U-shaped fixture at an oblique angle to the longitudinal direction of the MRI bed unit. In an embodiment, the helmet unit comprises two U-shaped fixture-mounting units, and one U-shaped fixture-mounting unit is constituted to mount the U-shaped fixture at an oblique angle to the longitudinal direction of the MRI bed unit, and the other U-shaped fixture-mounting unit is constituted to mount the U-shaped fixture at a right angle to the longitudinal direction of the MRI bed unit. In another embodiment, two U-shaped fixture-mounting units are each constituted to mount the U-shaped fixture at an oblique angle to the longitudinal direction of the MRI bed. In another embodiment, two U-shaped fixture-mounting units are each constituted to mount the U-shaped fixture at a right angle to the longitudinal direction of the MRI bed.

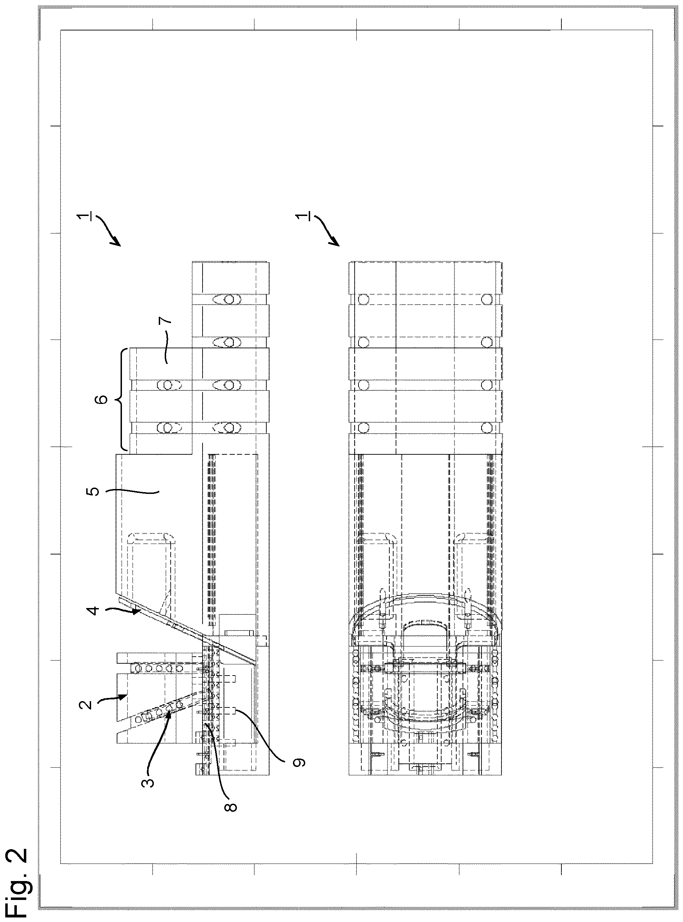

When a U-shaped fixture is to be mounted at an oblique angle to the longitudinal direction of the standard MRI bed, for example, the angle shown in Figure 2 can be employed, although the angle is not limited thereto. In an embodiment, and when a U-shaped fixture is to be mounted at an oblique angle to the longitudinal direction of the MRI bed, and when the direction perpendicular to the longitudinal direction of the MRI bed is designated as 90 degrees, the longitudinal direction of the nose side of the animal is designated as 0 degrees, and the longitudinal direction of the tail side of the animal is designated as 180 degrees, the oblique angle can be 45 to 85 degrees, 50 to 80 degrees, 55 to 75 degrees, 60 to 74 degrees, 65 to 73 degrees, 66 to 72 degrees, 67 to 71 degrees, or 68 to 70 degrees, such as 70 degrees, relative to the degrees designated above, although the angle is not limited thereto. When the U-shaped fixture is mounted at an oblique angle, which is smaller than 90 degrees, to the longitudinal direction of the MRI bed, this is expressed as "the U-shaped fixture is mounted at a sharp angle to the longitudinal direction of the MRI bed" herein for the convenience of description. In another embodiment, when the U-shaped fixture is to be mounted at an oblique angle to the longitudinal direction of the MRI bed, and when the direction perpendicular to the longitudinal direction of the MRI bed is designated as 90 degrees, the longitudinal direction of the nose side of the animal is designated as 0 degrees, and the longitudinal direction of the tail side of the animal is designated as 180 degrees, the oblique angle can be 95 to 135 degrees, 100 to 130 degrees, 105 to 125 degrees, 106 to 120 degrees, 107 to 115 degrees, 108 to 114 degrees, 111 to 113 degrees, or 110 to 112 degrees, such as 110 degrees, relative to the degrees designated above, although the angle is not limited thereto. When the U-shaped fixture is mounted at an oblique angle, which is larger than 90 degrees, to the longitudinal direction of the MRI bed, it is expressed as "the U-shaped fixture is mounted at an obtuse angle to the longitudinal direction of the MRI bed" herein for the convenience of description.

In a standard functional MRI bed, and with regard to a helmet unit equipped with at least two U-shaped fixture-mounting units, when at least one U-shaped fixture-mounting unit (a first U-shaped fixture) is mounted at an oblique angle to the longitudinal direction of the MRI bed, and at least one U-shaped fixture-mounting unit (a second U-shaped fixture) is mounted at a right angle to the longitudinal direction of the MRI bed, the first U-shaped fixture can be mounted at a sharp angle to the longitudinal direction of the MRI bed. When at least two U-shaped fixture-mounting units are each constituted to mount the U-shaped fixture at an oblique angle to the longitudinal direction of the MRI bed, in an embodiment, at least one U-shaped fixture (the first U-shaped fixture) can be mounted at a sharp angle, and at least one U-shaped fixture (the second U-shaped fixture) can be mounted at an obtuse angle. In such a case, dimensions of the helmet can be adequately designed to restrain the anterior region of the head of the subject animal with the first U-shaped fixture and to restrain the posterior region of the head with the second U-shaped fixture. In an embodiment, dimensions of the helmet can be designed based on the average head size of the subject animal. In another embodiment, dimensions of the helmet can be designed to be larger than the average head size of the subject animal, and the U-shaped fixture can be adjusted in accordance with the animal that is actually subjected to measurement.

In the standard functional MRI bed according to the present invention, the animal subjected to measurement is placed in the sphinx position, and the head is then immobilized with the U-shaped fixture. The level (height) of the U-shaped fixture to be mounted can be adjusted with the use of, for example, 2, 4, 6, or 8 adjust screws. In an embodiment, the functional MRI bed according to the present invention is preferably adjusted in a manner such that the head of the marmoset would not move (in the superior/inferior, right/left, and anterior/posterior directions) by a distance of 0.5 mm or longer.

### Improved functional MRI bed

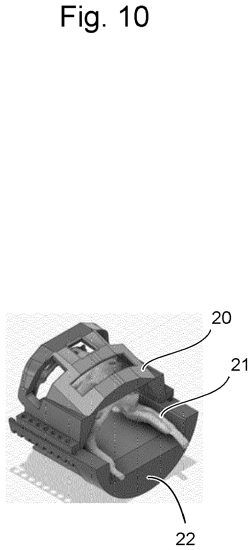

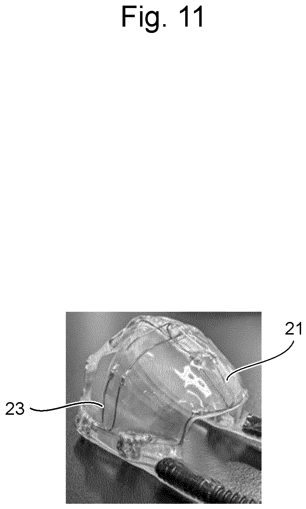

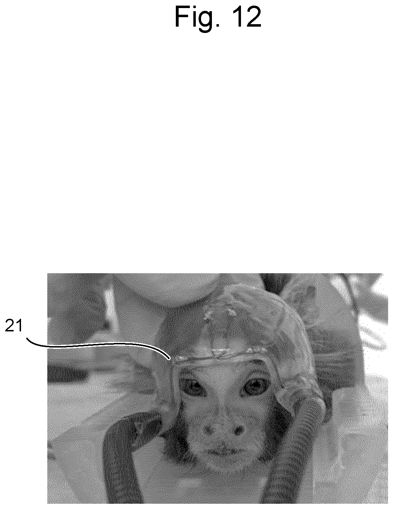

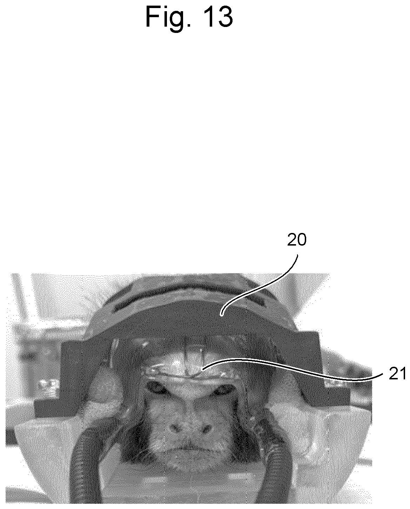

A standard functional MRI bed is equipped with a U-shaped fixture. After the head of the animal is immobilized with the U-shaped fixture, the entire functional MRI bed as shown in Figure 3 is inserted into the space inside the coil, and then MRI measurement is performed. In contrast, the improved functional MRI bed does not involve the use of a U-shaped fixture. The improved functional MRI bed is equipped with a helmet unit having an inner helmet and an outer helmet. Figure 10 shows an example of the inner helmet and the outer helmet, and Figure 13 shows an example of the helmet mounted on an animal. The inner helmet can comprise a coil mounted thereon. A coil-mounted inner helmet may be referred to as a "coil-integrated inner helmet." The dimensions and the configuration of the inner helmet can be designed in accordance with the dimensions and the configuration of the head of the subject laboratory animal (see, for example, Figure 11 and Figure 12). In an embodiment, the dimensions and the configuration of the inner helmet can be for general purposes. General purpose refers to the helmet not being limited to a particular individual animal, but being capable of mounting on a plurality of individual animals, and being capable of performing functional MRI measurement on the plurality of individual animals. With the use of the helmet unit having the inner helmet and the outer helmet, body movement in the Z-axis direction; i.e., the longitudinal direction, can be suppressed. Further, in a particular embodiment, the inner helmet can be immobilized with the aid of sponges or the like. Thus, body movement of the animal in the X-axis direction and in the Y-axis direction; i.e., the right/left and superior/inferior directions, can be reduced.

Unless specified otherwise, non-magnetic materials are used for the functional MRI bed according to the present invention. In an embodiment, the functional MRI bed according to the present invention can be made of, for example, plastic, acrylic, or resin materials. In an embodiment, a screw to immobilize a U-shaped fixture is preferably made of a non-magnetic material including resin materials. In an embodiment, a screw can be a pan-head screw with the head diameter larger than the thread diameter. In another embodiment, a screw can be a so-called "set screw" with the head diameter equivalent to or smaller than the thread diameter. In an embodiment, for example, use of a set screw enables the functional MRI bed to be readily inserted into the MRI apparatus. In an embodiment, an inner helmet can be made of ultraviolet curable resin. For example, the main frame part of the inner helmet can be prepared by 3-D printing, a coil can be applied thereto, and ultraviolet curable resin can then be overlaid thereon. In an embodiment, the coil is provided inside (the animal side of) the inner helmet. In another embodiment, the coil is provided outside the inner helmet.

In an embodiment, the functional MRI bed according to the present invention is equipped with a means for immobilizing the shoulder, neck, and waist region of an animal. In an embodiment, the functional MRI bed according to the present invention is equipped with a configuration to accommodate the forelimbs; i.e., a pocket. With this, the animal can be placed in the sphinx position, and the physical stress can be reduced.

In an embodiment, the functional MRI bed according to the present invention can be entirely or partially prepared by 3-D printing or injection molding. In an embodiment, the inner helmet can be prepared by 3-D printing or injection molding.

The functional MRI bed according to the present invention can be used for small animals and large animals. In an embodiment, the functional MRI bed according to the present invention is for marmosets. In another embodiment, the functional MRI bed according to the present invention may be for laboratory animals or pet animals, such as small animals including mice, rats, guinea pigs, and macaques. In another embodiment, the functional MRI bed according to the present invention may be for newborn humans.

In an embodiment, the present invention provides a method of functional MRI measurement of an animal using the functional MRI bed according to the present invention. For example, an animal is first positioned on a functional MRI bed. The animal is positioned inside a tunnel unit and placed in the sphinx position by accommodating the forelimbs in the pocket. Subsequently, a collar is mounted to immobilize the neck. Subsequently, the chin of the animal is placed on a chin rest and the helmet is mounted on the animal in that state. When a standard functional MRI bed is to be used, a U-shaped fixture is inserted into the U-shaped fixture-mounting unit, and the U-shaped fixture is then adjusted. In such a case, the level of the U-shaped fixture is adjusted in a manner such that the head of the animal would not substantially move, for example, the head of the animal would not move by a distance of 0.5 mm or longer in the superior/inferior, right/left, and anterior/posterior directions, and the U-shaped fixture is then immobilized with a screw. When an improved MRI bed is to be used, a coil-mounted inner helmet is first mounted on the animal. According to need, the both sides of the inner helmet may be stabilized with sponges, so as to prevent the animal from moving. The outer helmet is then mounted. The hindlimbs or the lower body can be immobilized with a zip tie or tape. Subsequently, the functional MRI bed on which the animal is immobilized is mounted on the MRI apparatus to perform functional MRI measurement.

A conventional or commercially available MRI apparatus can be used. In an embodiment, a magnetic flux density of the MRI apparatus can be 7 to 14, 7 to 11.7, or 7 to 9.4 Tesla, such as 7.0, 9.4, 11.7, or 14 Tesla, although the magnetic flux density is not limited thereto. In an embodiment, the bore diameter of the MRI apparatus can be 15 cm or smaller, 14 cm or smaller, 13 cm or smaller, 12 cm or smaller, 11 cm or smaller, 10 cm or smaller, 9 cm or smaller, or 8 cm or smaller, such as 7 cm to 15 cm, 7.2 cm to 14 cm, or 7.2 cm to 10 cm. The inner diameter is the diameter of the inside of the bore. The functional MRI bed according to the present invention can be designed to have dimensions compatible with the MRI apparatus. The functional MRI bed can be designed using CAD, 3D CAD, and the like. Use of the functional MRI bed according to the present disclosure enables functional MRI measurement to be performed at a high resolution using an MRI apparatus having a standard magnetic field intensity (e.g., 7 Tesla, 6 Tesla, or 5 Tesla). In an embodiment, for example, use of the functional MRI bed according to the present disclosure enables functional MRI measurement to be performed at a resolution equivalent to that achieved by conventional MRI measurement performed using an MRI apparatus having a high magnetic field intensity (e.g., 10 Tesla, 11 Tesla, or 11.9 Tesla). In an embodiment, the functional MRI bed according to the present disclosure can yield a resolution of 0.6 micron/7 Tesla or higher; i.e., 0.086 micron/ Tesla or higher, per unit magnetic flux density in the case of a marmoset. This indicates that a resolution per unit magnetic flux density is higher than that achieved by, for example, a conventional method that achieves the same resolution at a magnetic field intensity of 11.9 Tesla.

The functional MRI bed according to the present invention does not involve the use of a head post, and is thus non-invasive. Non-invasive refers to surgical treatment or surgical operation not being required. The functional MRI bed according to the present invention can be used for small-bore MRI to large-bore MRI. In the standard functional MRI bed according to the present invention, a U-shaped fixture can be adjusted in accordance with the dimensions and the configuration of the head of an individual subjected to functional MRI measurement and, therefore, it is not necessary to design a helmet for each animal subjected to measurement, and it is possible to use a helmet for general purpose for different animals. In addition, the improved functional MRI bed according to the present disclosure can be used for general purposes for different individuals or different animal species without the need of surgical treatment or surgical operation. The term "general purpose" used herein refers to a situation in which the same helmet or bed can be used for different individuals or different animal species. In other words, conventional MRI beds were customized for certain individual animals; that is, it was necessary to design a first helmet for an individual animal and to design a second helmet for another individual animal. In contrast, the functional MRI bed according to the present invention can be used for general purposes for different individual animals. In addition, the present invention does not require invasive treatment on the head of the subject animal and, therefore, the same individual animal can be analyzed or examined continuously for a long period of time, such as over months or years. In addition, the standard functional MRI bed and the improved functional MRI bed according to the present disclosure enable functional MRI measurement to be performed without anesthesia. This can be used, for example, to measure the brain of an awake individual animal in a resting state. Further, based on the brain activity measured in a resting state, the default mode network can be extracted by analysis. Further, a stimulus can be applied to a subject by, for example, showing an image, and the brain activity can then be measured.

### Examples

The present invention is described in greater detail with reference to the following examples, although the present invention is not limited thereto.

### Example 1: Standard functional MRI bed

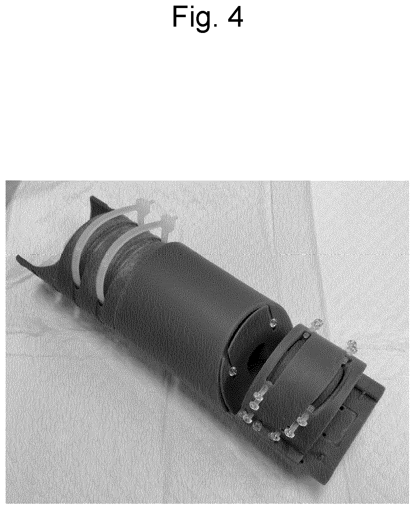

The functional MRI bed shown in Figure 3 was designed using 3D CAD and prepared using a 3D printer. The functional MRI bed was made of resin materials. Commercially available nylon zip ties were used to immobilize the lower body of an animal. Polycarbonate screws were used to immobilize the U-shaped fixture. Figure 4 shows a photograph of the functional MRI bed prepared.

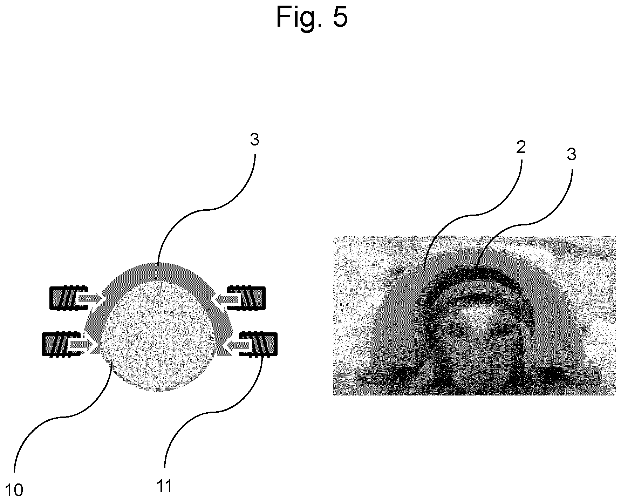

In this example, a 5-year-old male marmoset was positioned in the tunnel unit and placed in the sphinx position by accommodating the forelimbs in the pocket. The collar was then mounted to immobilize the neck. Subsequently, the chin of the marmoset was placed on a chin rest, and the helmet was mounted on the marmoset. Subsequently, the U-shaped fixtures were inserted into the U-shaped fixture-mounting unit, and the U-shaped fixtures were immobilized with screws to immobilize the head of the marmoset. In this example, of the two U-shaped fixtures, the U-shaped fixture on the anterior head side of the animal was mounted at an oblique angle to the longitudinal direction of the bed, and the U-shaped fixture on the posterior head side of the animal was mounted at a right angle to the longitudinal direction of the bed. The lower body was immobilized with a zip tie. Figure 5 shows a schematic view and a photograph of the immobilized head. Subsequently, the functional MRI bed was mounted on a functional MRI apparatus, and functional MRI measurement was performed. MRI was performed using PharmaScan 70/16 (Bruker) with the following parameters: repetition/echo time: 1500/15 ms; field of view: 38.4 × 38.4 mm; image matrix: 96 × 96, slice thickness: 1 mm, 21 slices; repetitions: 200.

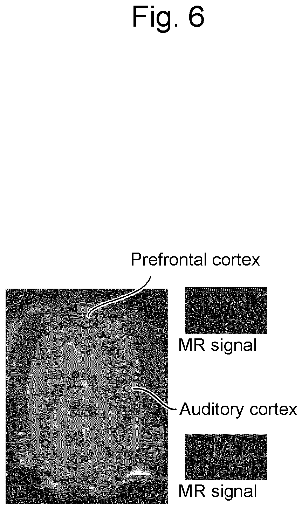

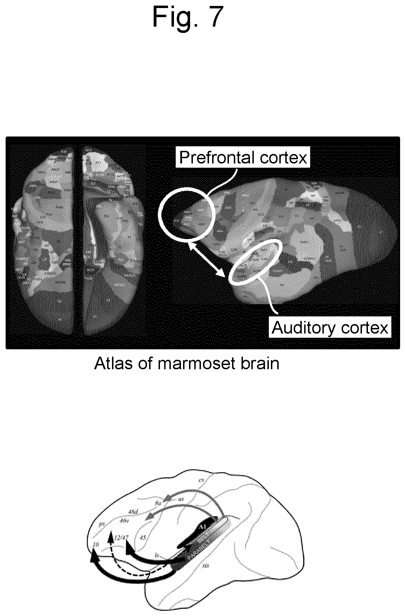

The brain activity of the marmoset when hearing a phee call; i.e., a contact call, was measured. Figure 6 shows the results. The results demonstrate that the activity of the auditory cortex is negatively correlated with the activity of the prefrontal cortex and that there is linkage between activities. Since it is known that there is a pathway through which the auditory cortex transmits information to the prefrontal cortex when a human or marmoset hears a sound (Figure 7), such activity may be appropriate activity between regions.

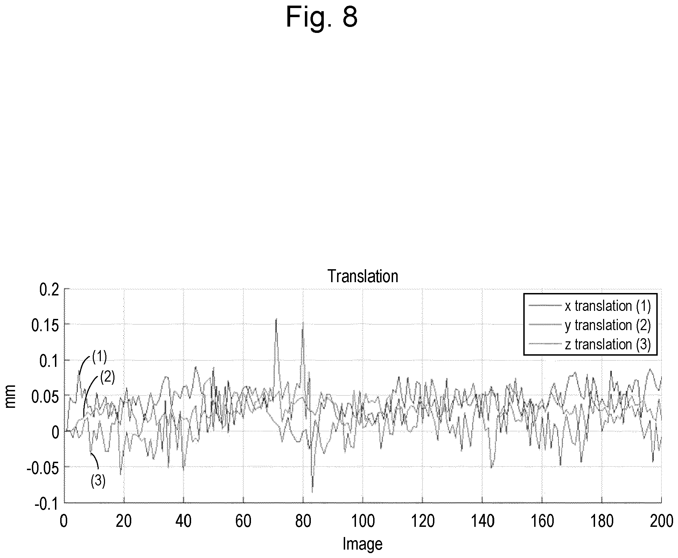

In order to obtain an image of the brain activity of the marmoset by functional MRI, a spatial resolution of 0.5 mm or less is necessary. If the subject laboratory animal moves by a distance of 0.5 mm or more, image processing cannot be performed, and the image becomes inappropriate. As such, it is necessary that the head of the marmoset does not move by a distance of 0.5 mm or more in the superior/inferior, right/left, and/or anterior/posterior directions. On the basis of the MRI data obtained when the animal heard a phee call, positional shifts of the head of the animal on planes perpendicular to the longitudinal direction of the bed (the x axis and the y axis) and in the longitudinal direction of the bed (the z axis) were analyzed. Figure 8 shows the results of analysis. The maximal shift on an axis was 0.23 mm, and, when the shift occurred, the shifts on the other axes were within 0.05 mm. In addition, the brain activity of the marmoset was effectively measured by using the produced functional MRI bed and, therefore, it was demonstrated that the functional MRI bed according to the present invention can be used for general purposes.

Figure 9 shows another example of the present invention. In this example, of the two U-shaped fixtures provided in the helmet, a U-shaped fixture on the face side of the animal (the anterior head side) is mounted at a sharp angle to the longitudinal direction of the bed, and the other U-shaped fixture on the posterior head side of the animal is mounted at an obtuse angle to the longitudinal direction of the bed (Figure 9, right).

The MRI bed according to the present invention is composed of radiolucent materials and, therefore, it can be used for CT imaging.

As described above, the brain activity of the marmoset was measured without mounting a skull head bar or head post on the head of the animal by surgical operation. The functional MRI bed according to the present invention can be used for a plurality of individuals by adjusting the U-shaped fixtures and can be thus used for general purposes.

### Example 2: Improved functional MRI bed

When the bore diameter of an MRI apparatus was relatively small (e.g., 10 cm or smaller), in some embodiments, it was not possible to acquire sufficient space while providing the receiver coil close to the subject animal to improve signal-to-noise ratio of the image. In order to overcome such problem, the present inventors provided a mechanism that would enable improved image quality and immobilization of the head of the subject animal by using the receiver coil as a fixture to immobilize the target of imaging. Use of such MRI apparatus (also referred to as an "improved MRI apparatus") enables the receiver coil to be provided in a narrow space. Awake MRI can also be measured. This mechanism enables observation of changes in the brain or organs before and after the administration of a particular drug.

### Improved MRI apparatus

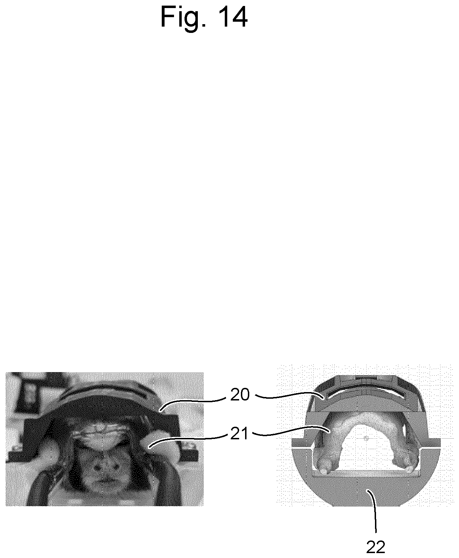

The head of the laboratory animal was immobilized with a dual structure of an inner helmet and an outer helmet. According to such configuration, the coil can be integrated into the inner helmet. Figure 11 and Figure 12 each show the coil-integrated inner helmet. First, the inner helmet was mounted on the animal, and the outer helmet was overlaid thereon to immobilize the head part. Figure 10 and Figure 13 each show a configuration comprising the outer helmet overlaid on the inner helmet. According to such configuration, it is possible to further immobilize the inner helmet with sponges or the like so as to reduce body movement of the animal (Figure 13 and Figure 14).

Subsequently, the animal on which the improved helmet was mounted was subjected to MRI measurement. The following equipment were used.
1) A 7.0T magnetic resonance imaging (MRI) apparatus for small animals (Biospec 70/16; Bruker) (the same as used in Example 1)
2) MRI coil
   - Receiver coil (inner helmet): a 4-channel phased-array coil (Takashima Seisakusho Co., Ltd.)
   - Transmission coil: 72-mm volume coil (Bruker)

The information of the MRI image is as described below.
- Imaging range: 38.4 × 38.4 × 21 (mm)
- Resolution: 0. 6 × 0.6 × 1 (mm)

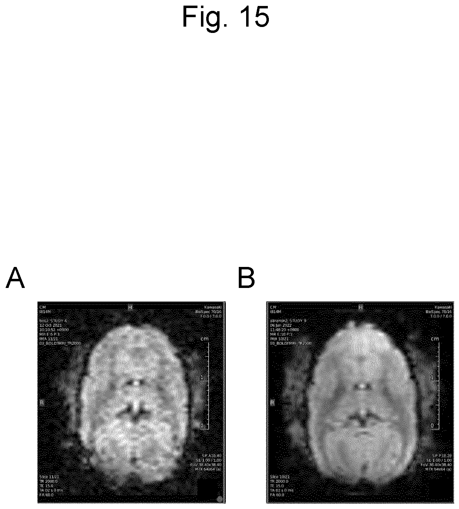

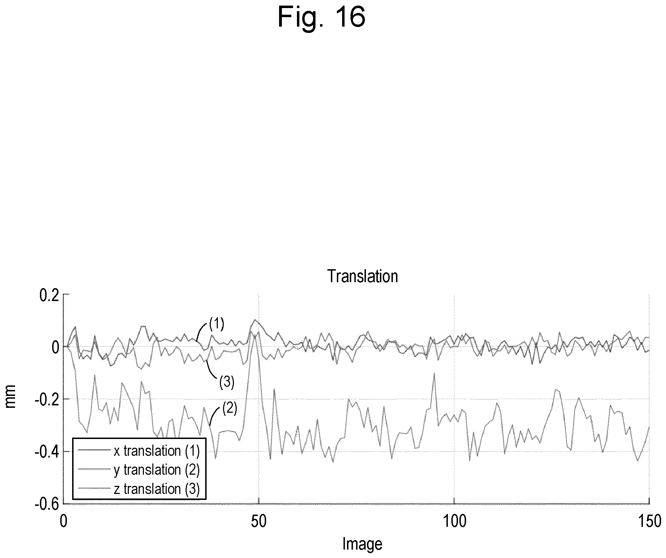

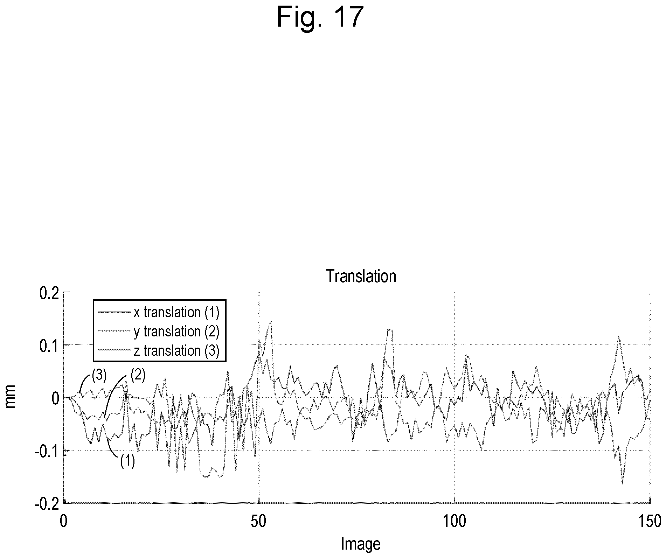

The results are shown in Figure 15, Figure 16, and Figure 17. Figure 15 shows the results obtained using the MRI bed of Example 1 at the left and the results obtained using the improved MRI bed of Example 2 at the right. Figure 16 shows the results obtained using the MRI bed of Example 1, and Figure 17 shows the results obtained using the improved MRI bed of Example 2. The results demonstrate that body movement of the animal can be suppressed with the use of the improved MRI bed at the level equivalent to the level achieved using the MRI bed of Example 1. When images were obtained by using the improved MRI bed, the signal-to-noise ratio was improved by approximately two fold, compared to that before the use thereof (Figure 15).

The inner helmet is first mounted on the animal, the animal is loaded on the MRI bed, and the outer helmet is then overlaid thereon to immobilize the head part. This enables obtaining clearer images, compared with images obtained by conventional techniques or by using the MRI bed of Example 1. Further, because the entire head of the animal is immobilized, use of U-shaped fixtures is not necessary, and the pain level inflicted on the animal can be reduced.

In addition, the improved MRI helmet was mounted on six different marmosets, and functional MRI measurement was performed on all six individuals. Conventional methods required the use of an expensive coil in accordance with the animal species or individual animal in each case of measurement. Thus, imaging was restricted from the aspect of cost. Further, when one coil was to be used on another individual animal, the coil would not necessarily be optimal due to differences in sizes or configurations of the imaging subjects, and this resulted in poor resolution. For example, the helmet described in Non-Patent Literature 2 was customized individually to be compatible with the individual animal. The method described in Non-Patent Literature 1 requires mounting a head bar on the skull by surgical operation and, therefore, such method could not be employed for general purposes and would inflict pain from surgical operation on the imaging subject animal. According to the configuration of the present disclosure, the cost would not be increased, and a plurality of animal species or a plurality of animal individuals can be subjected to imaging by using a single coil. In addition, the configuration of the present disclosure does not require surgical operation and this can eliminate surgical risk factors and pain inflicted on individual animals.

Figure 15 shows an awake MRI image of the head obtained by using the improved MRI bed (bore diameter: 72 mm, left) and an MRI image of the head under anesthesia obtained by using a coil with a diameter suitable for imaging of rats (bore diameter: 38 mm, right). Despite the images being obtained using an MRI bed with a larger bore diameter, no coarse noises were observed in the fMRI images or the T2-weighted images, and equivalent signal-to-noise ratios were achieved both on the right side and the left side.

### Example 3: Functional MRI measurement with a rat

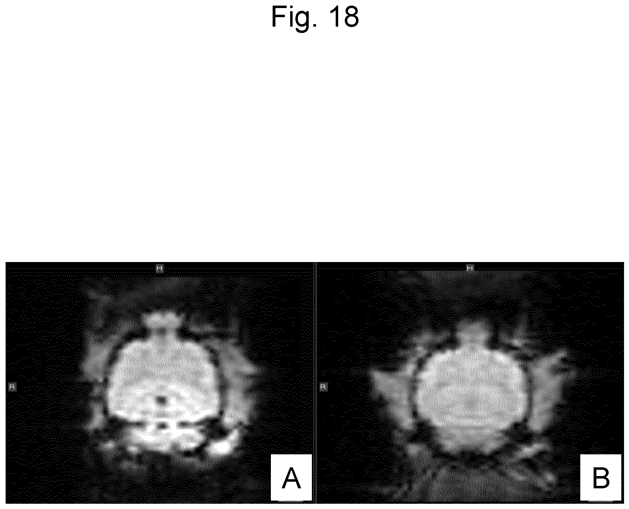

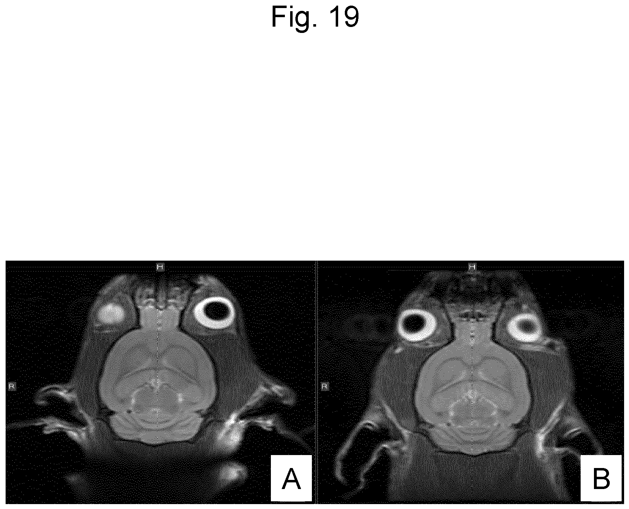

The improved MRI helmet of Example 2 per se was used on a rat without any modifications. The head of the rat wearing the inner helmet was immobilized with sponges and with the outer helmet. Figure 18 A shows an image of an awake rat wearing the helmet of the present invention, Figure 18 B shows an image obtained using fixtures using a conventional single-purpose coil for the rat head, and the resolution of the former was equivalent to that of the latter. Figure 19 shows a T2-weighted image often used in MRI imaging. Figure 19 A shows an image of an awake rat wearing the helmet of the present invention, and Figure 19 B shows an image of an anesthetized rat obtained using the single-purpose coil for the rat as shown in Figure 18 B. According to the present invention, the image resolution equivalent to that achieved for an anesthetized rat using a conventional coil was obtained under an awake state. Use of the helmet according to the present invention enabled functional MRI measurement on a rat. According to the configuration of the present disclosure, a plurality of animal species can be subjected to imaging with the use of a single coil.

### Example 4: Awake CT imaging of marmoset

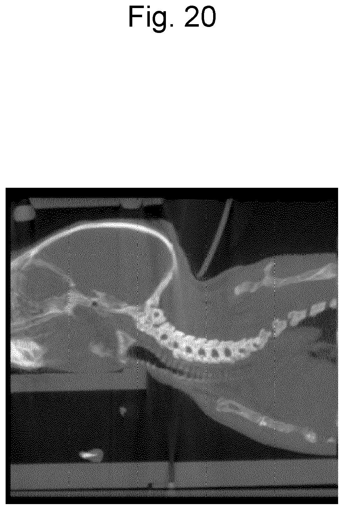

CT was performed on a marmoset using the standard functional MRI bed. As with the case of MRI, the head of the marmoset was immobilized with the helmet and the U-shaped pins, and awake CT was performed as shown in Figure 20.

### Example 5: Eye tracking

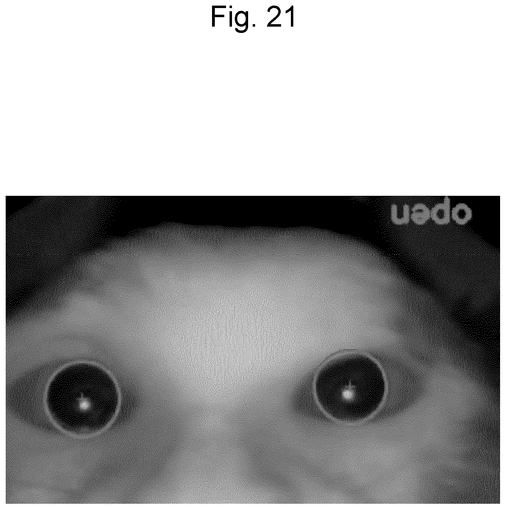

In the case of the standard functional MRI bed, the head is immobilized and, therefore, it is possible to observe the face of the subj ect from the front. When the marmoset was subjected to MRI imaging in the sphinx position, the marmoset was video-recorded from the front of the face thereof, and the iris regions were detected to perform eye tracking simultaneously with MRI imaging. Figure 21 shows an image of eye tracking by detecting the iris regions.

### Industrial Applicability

The functional MRI bed according to the present invention can be used for MRI measurement and CT imaging of a laboratory animal.

The present description cites numerous documents including manufacturers' instructions. While the disclosures of such documents are not regarded as being related to patentability of the present invention, such disclosures are incorporated herein by reference in their entirety. More specifically, all of the reference documents are incorporated herein by reference as in the case in which each of the documents is specifically and individually indicated to be incorporated herein by reference.

### Reference Signs List

1: Functional MRI bed
2: Helmet
3: U-shaped fixture
4: Collar
5: Tunnel unit
6: Corset
7: Sponge
8: Chin rest
9: Pocket
10: Animal head
11: Screw
20: Outer helmet
21: Inner helmet
22: Improved MRI bed (bed body)
23: Coil

All publications, patents, and patent applications cited herein are incorporated herein by reference in their entirety.

## Claims

1. A general-purpose functional magnetic resonance imaging (MRI) bed equipped with a bed unit, a collar unit, and a helmet unit,
wherein
the bed unit is constituted to hold a laboratory animal in the sphinx position,
the helmet unit is to immobilize the head of the laboratory animal, so as to perform functional MRI measurement, and the helmet unit comprises an inner helmet comprising a coil integrated therein and an outer helmet, and
the collar unit is to restrain the neck and the shoulder of the laboratory animal.

2. The general-purpose functional MRI bed of claim 1, wherein the bed, the helmet, and the collar are made of non-magnetic materials.

3. The general-purpose functional MRI bed of claim 1 or 2, which is used for a marmoset, mouse, rat, macaque, guinea pig, or newborn human.

4. A general-purpose functional MRI bed equipped with a bed unit, a helmet unit, a collar unit, and a U-shaped fixture,
wherein
the bed unit is constituted to hold a laboratory animal in the sphinx position,
the helmet unit is to immobilize the head of the laboratory animal, so as to perform functional MRI measurement, and the helmet unit comprises a U-shaped fixture-mounting unit to mount a U-shaped fixture,
the collar unit is to restrain the neck and the shoulder of the laboratory animal, and
the U-shaped fixture is adjustable in accordance with the dimensions and the configuration of the head of the laboratory animal subjected to functional MRI measurement.

5. The general-purpose functional MRI bed of claim 4, wherein the helmet unit comprises two or more U-shaped fixture-mounting units to mount U-shaped fixtures.

6. The general-purpose functional MRI bed of claim 4, wherein the U-shaped fixture-mounting unit is constituted to mount the U-shaped fixture at a right angle to the longitudinal direction of the MRI bed unit or to mount the U-shaped fixture at an oblique angle to the longitudinal direction of the MRI bed unit; or
(i) the general-purpose functional MRI bed of claim 5, wherein the U-shaped fixture-mounting unit is constituted to mount at least one U-shaped fixture at an oblique angle to the longitudinal direction of the MRI bed unit and to mount at least another U-shaped fixture at a right angle to the longitudinal direction of the MRI bed unit,
(ii) the general-purpose functional MRI bed of claim 5, wherein the U-shaped fixture-mounting unit is constituted to mount at least one U-shaped fixture at an oblique angle to the longitudinal direction of the MRI bed unit and to mount at least another U-shaped fixture at an oblique angle to the longitudinal direction of the MRI bed unit, or
(iii) the general-purpose functional MRI bed of claim 5, wherein the U-shaped fixture-mounting unit is constituted to mount at least one U-shaped fixture at a right angle to the longitudinal direction of the MRI bed unit and to mount at least another U-shaped fixture at a right angle to the longitudinal direction of the MRI bed unit.

7. The general-purpose functional MRI bed of claim 6 dependent on claim 4, wherein the U-shaped fixture-mounting unit is constituted to mount a U-shaped fixture at a sharp angle to the longitudinal direction of the MRI bed unit; or
(i) the general-purpose functional MRI bed of claim 6 dependent on claim 5, wherein the U-shaped fixture-mounting unit is constituted to mount at least one U-shaped fixture at a sharp angle to the longitudinal direction of the MRI bed unit and to mount at least another U-shaped fixture at a right angle to the longitudinal direction of the MRI bed unit, or
(ii) the general-purpose functional MRI bed of claim 6 dependent on claim 5, wherein the U-shaped fixture-mounting unit is constituted to mount at least one U-shaped fixture at a sharp angle to the longitudinal direction of the MRI bed unit and to mount at least another U-shaped fixture at an obtuse angle to the longitudinal direction of the MRI bed unit.

8. The general-purpose functional MRI bed of any one of claims 4 to 7, wherein the bed, the helmet, the collar, the U-shaped fixture, and a screw to immobilize the U-shaped fixture are made of non-magnetic materials.

9. The general-purpose functional MRI bed of any one of claims 4 to 8, which is used for a marmoset.

10. A functional MRI bed system equipped with the general-purpose functional MRI bed of any one of claims 4 to 9 and an MRI apparatus.

11. A method of functional MRI measurement for an animal, comprising using the general-purpose functional MRI bed of any one of claims 4 to 9 or the functional MRI bed system of claim 10.

12. The method of claim 11, comprising measuring the brain in a resting state.

13. The method of functional MRI measurement of an animal of claim 11, wherein an imaging device is provided in front of the face of the subject animal of imaging, and the MRI measurement is performed simultaneously with eye tracking.
